(19) Europäisches Patentamt / European Patent Office / Office européen des brevets

(11) **EP 3 106 090 A1**

(12) **EUROPEAN PATENT APPLICATION**

(43) Date of publication:
**21.12.2016 Bulletin 2016/51**

(21) Application number: **15172198.2**

(22) Date of filing: **15.06.2015**

(51) Int Cl.:
*A61B 5/087* (2006.01)     *A61M 15/00* (2006.01)
*A61M 15/08* (2006.01)     *A61M 16/00* (2006.01)
*A61M 16/04* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(71) Applicants:
• **CHIESI FARMACEUTICI S.p.A.**
**43122 Parma (IT)**

• **Politecnico Di Milano**
**20133 Milano (IT)**

(72) Inventors:
• **DELLACA', Raffaele**
**43122 Parma (IT)**
• **MILESI, Ilaria**
**43122 Parma (IT)**

(74) Representative: **Zerbi, Guido Maria**
**Murgitroyd & Company**
**Piazza Borromeo, 12**
**20123 Milano (IT)**

(54) **SYSTEM FOR EFFECTIVE BREATH-SYNCHRONIZED DELIVERY OF MEDICAMENT TO THE LUNGS**

(57) The method and system according to preferred embodiments of the present invention allows an effective breath-synchronized delivery of atomized liquid medicament (e.g. a pulmonary surfactant) to the patient's lungs. According to a preferred embodiment, the method of the present invention provides an efficient delivery of the aerosol medicament, controlling the behavior of the infusion pump to make the rising and falling time faster even though the intrinsic time constant of the system is long. Additionally, in an embodiment of the present invention, at the same time the information about the breathing activity contained either directly on the surfactant line or stored in the controller action can be used to extrapolate the breathing pattern.

*Fig. 17*

EP 3 106 090 A1

**Description**

Field of **technology**

The present invention relates to the field of retropharyngeal instillation of medicament and particularly to a method and system for the administration of a pulmonary surfactant by atomization with a breath/synchronized delivery.

**Background of the invention**

Preterm infants are prone to develop IRDS (Infant Respiratory Distress Syndrome) because of generalized lung immaturity. Nowadays, clinical management of preterm RDS infants mostly relies on 1) providing respiratory support and 2) administering exogenous pulmonary surfactant. The current most widely accepted approach for providing respiratory support to newborns is focused on avoiding invasive mechanical ventilation and intubation in favour of non-invasive respiratory support approaches such as nasal continuous positive airway pressure (CPAP), nasal intermittent positive pressure ventilation (NIPPV) or high flow nasal cannula (HFNC) whenever mechanical ventilation is not strictly necessary. Even if these approaches showed good results, they are not directly addressing surfactant deficiency and a significant number of newborns still requires exogenous surfactant therapy. Pulmonary surfactant administration is currently performed by bolus administration, as this is the only clinically proven effective administration approach. Unfortunately, bolus administration requires intubation, a complex and invasive procedure that might be associated to several side-effects. Moreover, bolus administration is often associated to hemodynamic and systemic fluctuations, due to the amount of liquid administered in the lungs and due to the sudden following reduction of lung resistance, that, in turn, are considered further potential risks for the babies.

Because of these implications, a great effort has been dedicated in finding alternative ways for administration of pulmonary surfactants. In particular, the administration by nebulization has been extensively studied. In more details, commercial nebulizer were inserted along the ventilator circuit. So far, the results of these trials are inconclusive, showing very poor surfactant deposition into the lungs.

In turn, poor deposition could be due to one or more of the following occurrences: 1) a significant amount of surfactant deposits along the ventilator connections, 2) if the patient is under CPAP, the surfactant that is not inspired deposits in the upper airways and is then swallowed by the newborn instead of achieving the lungs, and 3) if the nebulizer is not synchronized with the neonate's breath, the surfactant nebulized during expiration is exhaled.

A possible alternative approach that could overcome these issues was proposed by Wagner et al. (Wagner MH, Amthauer H, Sonntag J, Drenk F, Eichstädt HW, Obladen M "Endotracheal surfactant atomization: an alternative to bolus instillation?" Crit Care Med. 2000; 28(7):2540). They used a modified tracheal tube with an atomizer inserted at the tip of the tube, which produced relatively big particles compared to typical nebulization (~100 $\mu$m) only during inspiration (manually synchronized by an operator). Despite big technological issues and despite the need for intubation, this approach showed to be effective and was further developed by Chiesi and Polytechnic of di Milano (see Patent Application WO2013/160129). The resulting implemented embodiment provides an improvement aiming at overcoming the limitations of previous approaches. In fact this device allows: 1) to deliver surfactant in the newborn's pharyngeal region during spontaneous breathing and 2) to maximize the quantity of surfactant delivered by synchronizing the atomization with the inspiratory breathing phase. The device disclosed in WO2013/160129 is schematically shown in Figure 1: it includes a small and flexible atomizing catheter that can be inserted in the pharyngeal region of the patient connected to an infusion pump that delivers the liquid medicament (for example surfactant) and source of compressed gas. The atomizing catheter includes a first channel where the liquid medicament flows to be conveyed to the patient's pharyngeal region and a second channel that conveys a pressurized flow of gas at the tip of the catheter. The pump moves the column of liquid medicament towards the tip of the catheter so that, when the medicament and the pressurized gas meet in the pharyngeal cavity, the liquid is broken into small particles causing atomization of the medicament and the delivery of the drug into the patient's lungs.

The system further includes a pressure sensor along the surfactant line for measuring a value indicative of the pressure in the patient pharyngeal cavity, such value being used to determine whether the patient is in an inspiration or in an expiration phase and to activate the pump only during the inspiratory phase. The pressure measured along the surfactant line will be the sum of two contributions: 1) the drop of pressure due to the medicament flow through the catheter and 2) the pressure swings due to the breathing activity.

Since the medicament lumen is very thin and the medicament could be viscous, the flow resistance associated to this channel can be very high. Moreover, as the liquid medicament has to be loaded into the system and, during this procedure, it is very likely that small air bubbles appears into the medicament line and these bubbles, being compressible, behave like hydraulic compliances. The coupling of high resistance and compliances leads to potentially high time constants that have two detrimental impacts on the operation of the system:

1) long rising time in the pressure of the medicament when the infusion pump is activated, leading to long delays from when the volumetric pump starts to when the surfactant flows at the tip of the catheter.

2) it prevents a prompt detection of the breathing phase needed to synchronize the delivery of surfactant in phase with breathing as the breathing signal can be delayed and masked by the motor activation.

In patent application WO2013/160129 the delivery of the medicament and the sensing of the pressure has been done with a single catheter lumen, but this is only one of the possible implementations. For instance, in co-pending patent application PCT/EP2014/072278 filed by the same Applicants, Chiesi and Polytechnic of Milano and not published yet, an alternative embodiment is disclosed embodiment with a separate channel dedicated to the sensing of the breath.

By adding a dedicated sensing line, it has been possible to overcome the issue due to the detection of the breathing, since the sensing line can be designed without the strict constrains of the atomizing catheter, but, on the other hand, issue related to delay in the delivery still remains.

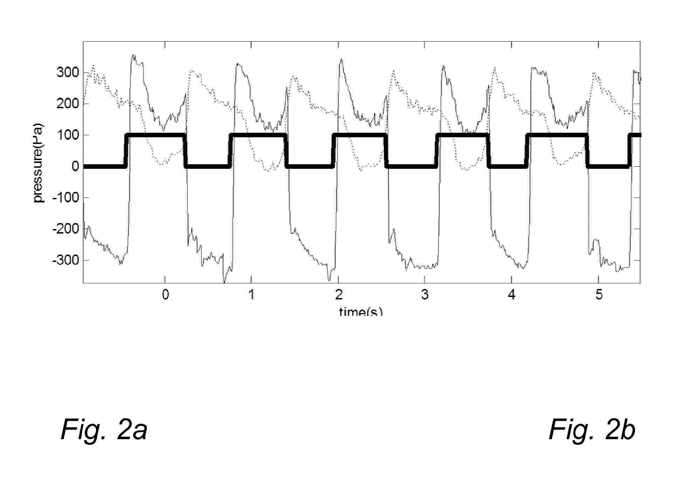

If special care is used to remove all air bubbles, the pressure signal recorded on the medicament line allows the detection of the respiratory phase. In Figure 2 it is possible to appreciate the reproducibility between the pressure measured along the surfactant line and the one recorded in the pharynx when a careful priming is performed and almost no bubbles are inserted into the circuit. The diagram on Figure 2 shows the comparison of the following values:

- pressure measured along the medicament (surfactant) line (solid line);
- pharyngeal pressure (dashed line);
- infusion pump motor activation signal (thick line).

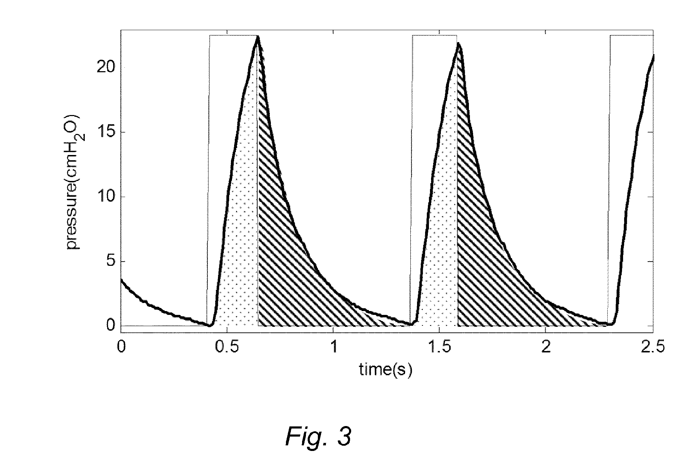

The diagram of Figure 2 shows the behaviour of the pressure measured along the medicament line during synchronized medicament delivery in case of ideal conditions, which are achievable by special care in removing gas bubbles from the medicament circuit, but which require a very skilled operator to perform the priming and a long time. Therefore, conditions of Figure 2, are unfortunately unlikely to happen in the real world. In case of bad priming of the circuit, which anyway means inserting a small amount of bubbles in the circuit in absolute terms, the time constant dramatically increases with a great impact on the system as shown in Figure 3.

The consequence of prolonged rising time is mainly that the time in which the medicament starts flowing in the atomizing tip is "delayed" compared to the time in which the infusion pump is turned on, compromising the possibility to administer the medicament only during the inspiratory phase and, therefore, reducing the total amount of medicament potentially deliverable to the patient's lung during each inspiration, leading to longer time for completing the treatment and waste of medicament. Moreover, when the pump is turned off, because of the long-time constant the flow does not immediately stop and part of the medicament is wasted as delivered during expiration.

In Figure 3, the thin line represents the motor activation and the expected pressure, the thick line represents the actual pressure (proportional to the flow) sensed on the surfactant line. The dotted area represents the amount of medicament that is properly administered during the inspiration, while the dashed area represents the medicament being wasted as delivered during the expiration of the patient.

Another side effect of the increased time constant is related to the detection of the breathing activity. If the rising time is too long, the breathing activity is totally masked by the activation of the motor, which has a biggest amplitude (it is equal to surfactant flow time catheter hydraulic resistance) compared to the breathing activity of the baby, preventing from detecting respiratory phase on the surfactant line.

Possible options to reduce the time constant of the system are:

1) Reduce the hydraulic resistance of the surfactant lumen. This can be done but if the surfactant lumen is too big, the airflow needed to atomize will be very high, because the surface contact between surfactant and compressed air is small compared to the cross section of the surfactant lumen. High airflow, such as higher than 1.5 litres per minute (LPM) are not compatible with the system, therefore this solution cannot be implemented.

2) Reduce the compliance of the system. This can be obtained by using rigid components, such as glass syringes and optimizing the mechanical components of the infusion pumps and above all, reducing the amount of bubbles in the surfactant with a careful priming, which is the preeminent cause for compliance. Unfortunately, this procedure is very time consuming and it is difficult to completely de-bubbling the system. Moreover the result of the priming procedure is quite unpredictable, making the working condition of the system difficult to define.

Both proposed adjustment options described above are rather heavy and time consuming and cannot completely solve the problem.

For all these reasons, an improved method and system for administering the exogenous pulmonary surfactant which is capable of compensating the delay in the delivery caused by the mechanical and hydraulic characteristics of the system would be greatly appreciated. Moreover, the possibility of implementing this method without requiring major changes in the mechanical and electronic components is another desirable feature.

**Objects of the invention**

It is an object of the present invention to overcome at least some of the problems associated with the prior art.

**Summary of the invention**

The present invention provides a method and system as set out in the accompanying claims.

According to one aspect of the present invention, we provide a system for delivering a liquid medicament to spontaneously breathing patients, comprising: i) a catheter adapted to reach the pharyngeal region of the patient, the catheter including at least a first channel being adapted to convey in the patient's pharyngeal region a flow of liquid medicament; ii) first pump means connected to a first end of the at least first channel, adapted to create a pressure which pushes the column of liquid medicament towards the second end of the at least first channel; iii) breathing detecting means, for measuring a value indicative of whether the patient is in an inspiration or in an expiration phase and wherein the first pump means are controlled by a microprocessor and are selectively activated only during inspiration phase, characterized in that the pressure applied to the first pump means is adapted by the microprocessor so that the delay induced by the hydraulic impedance of the system is substantially counterbalanced. The medicament can be e.g. pulmonary surfactant.

In a preferred embodiment of the present invention breathing detecting means includes pressure detective means, for measuring a value indicative of the pressure in the patient pharyngeal cavity, such value being used to determine whether the patient is in an inspiration or in an expiration phase.

According to a first possible option the pressure detecting means includes: iv) a pressure sensor connected to the first channel for measuring a value indicative of the pressure on the flow of the liquid medicament.

In a second possible option (alternative to the first option above) the pressure detecting means includes: iv) a second channel adapted to convey in the patients pharyngeal region a flow of a water solution; v) second pump means connected to a first end of the second channel, adapted to create the flow of water solution; vi) a pressure sensor connected to the second channel for measuring a value indicative of the pressure on the flow of water solution.

In any case the pressure detecting means could be connected and provide feedback to the microprocessor and the microprocessor estimates the hydraulic impedance according to measured values received from the pressure detecting means.

In a possible embodiment the hydraulic impedance is calculated as a function of the difference between the measured value received from the pressure detecting means and a predetermined expected value. Possibly the increase of the initial pressure is determined according to predetermined values. Such predetermined values can be stored in a lookup table accessible by the microprocessor.

In a preferred embodiment the catheter includes at least one dedicated gas channel adapted to convey in the patient's pharyngeal region a pressurized flow of gas, the system further comprising: gas pump means connected to a first end of the gas channel, adapted to create the flow of pressurized gas; so that when the column of liquid medicament and the pressurized gas meet in the pharyngeal cavity, the liquid column is broken into a plurality of particles causing the atomized medicament to be delivered into the patient's lungs.

Preferably, the aerosol medicament comprises an exogenous pulmonary surfactant, e.g. selected from the group consisting of modified natural pulmonary surfactants (e.g. poractant alfa), artificial surfactants, and reconstituted surfactants.

Also, in a preferred embodiment, the pressurized gas includes air or oxygen.

A still further aspect of the present invention provides a computer program for controlling the above described method.

The method and system of the present invention provides an efficient delivery of the aerosol medicament, controlling the behavior of the infusion pump to make the rising and falling time faster even though the intrinsic time constant of the system is long. Additionally, in an embodiment of the present invention, at the same time the information about the breathing activity contained either directly on the surfactant line or stored in the controller action can be used to extrapolate the breathing pattern. The method and system of the present invention provides several advantages including the use of components which are already familiar to the hospital personnel, e.g. catheters and disposable pressure sensors; all the part in contact with the pulmonary surfactant and the patient are low cost and disposable, granting for hygienically and safe treatments, which is particularly important when the patient is a pre-term neonate.

Brief description of the drawings

Reference will now be made, by way of example, to the accompanying drawings, in which:

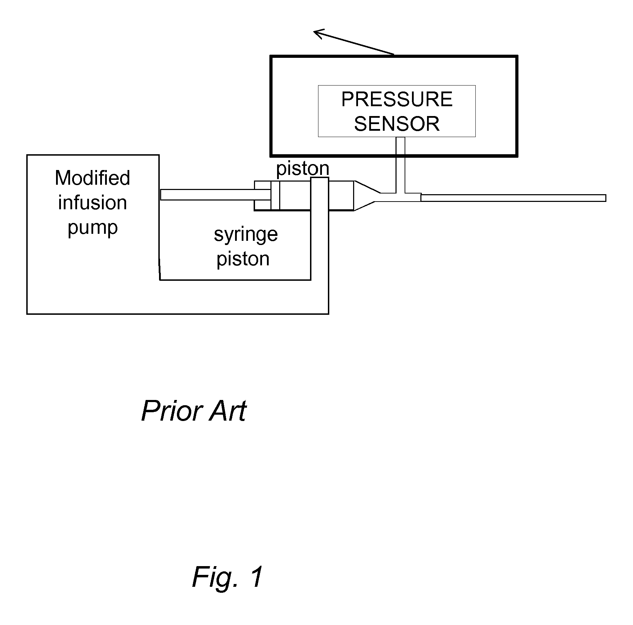

Figure 1 is a schematic diagram of one of the possible systems of the prior art;

Figures 2-3 show the behavior of the pressure measured along the medicament line during synchronized medicament delivery in ideal conditions (Fig.2) and in real world cases (Fig. 3) according to the prior art system of Fig.1;

Figure 4 shows a schematic mathematical representation of a system according to a preferred embodiment of the present invention;

Figure 5 is a schematic block diagram of a system according to a preferred embodiment of the present invention;

Figure 6 is a schematic diagram of the close loop control system according to a preferred embodiment of the present invention;

Figure 7 schematically represents a comparison between the behaviors of the Open loop control system vs Closed loop control system;

Figure 8 shows the results after the tuning obtained with an implementation of a system according to a preferred embodiment of the present invention;

Figure 9 shows a diagram representing the flow and the flow needed to produce the pressure measured in case of bubble of 0.2ml;

Figure 10 shows a schematic representation of a system according to a second embodiment of the present invention;

Figure 11 is a picture representing an implementation of a system according to a second embodiment of the present invention;

Figure 12 represents the result obtained with a system according to a second embodiment of the present invention;

Figure 13 schematically represents the mathematical description of a system according to a second embodiment of the present invention, while Figure 14 is a block diagram of the same system;

Figures 15-27 are schematic representations of systems, block diagrams, results and performances of third, fourth and fifth embodiments of the present invention.

## Definitions

The tem "liquid medicament" encompasses any medicament wherein the active ingredient is dissolved or suspended in the liquid medium, preferably suspended. The terms "neonates" and "newborns" are used as synonymous to identify very young patients, including pre-term babies having a gestational age of 24 to 36 weeks, more particularly between 26 and 32 weeks.

With the term "pulmonary surfactant" it is meant an exogenous pulmonary surfactant administered to the lungs that could belong to one of the following classes:

i) "modified natural" pulmonary surfactants which are lipid extracts of minced mammalian lung or lung lavage. These preparations have variable amounts of SP-B and SP-C proteins and, depending on the method of extraction, may contain non-pulmonary surfactant lipids, proteins or other components. Some of the modified natural pulmonary surfactants present on the market, like Survanta™ are spiked with synthetic components such as tripalmitin, dipalmitoylphosphatidylcholine and palmitic acid.

ii) "artificial" pulmonary surfactants which are simply mixtures of synthetic compounds, primarily phospholipids and other lipids that are formulated to mimic the lipid composition and behavior of natural pulmonary surfactant. They are devoid of pulmonary surfactant proteins;

iii) "reconstituted" pulmonary surfactants which are artificial pulmonary surfactants to which have been added pulmonary surfactant proteins/peptides isolated from animals or proteins/peptides manufactured through recombinant technology such as those described in WO 95/32992 or synthetic pulmonary surfactant protein analogues such as those described in WO 89/06657, WO 92/22315, and WO 00/47623.

The term "non-invasive ventilation (NIV) procedure defines a ventilation modality that supports breathing without the need for intubation.

## Detailed description of preferred embodiments

With reference to the accompanying figures an implementation of the method and system according to a preferred embodiment of the present invention is illustrated. In the example here discussed we addressed the problem of delivering the right amount of atomized medicament to a patient: in particular we administrated a pulmonary surfactant to a patient population e.g. a preterm neonate. The utilized pulmonary surfactant is poractant alfa, formulated as 80 mg/ml aqueous suspension and commercially available as Curosurf® from Chiesi Farmaceutici SpA.

However, any pulmonary surfactant currently in use, or hereafter developed for use in respiratory distress system and other pulmonary conditions could be suitable for use in the present invention. These include modified natural, artificial and reconstituted pulmonary surfactants (PS).

Current modified natural pulmonary surfactants include, but are not limited to, bovine lipid pulmonary surfactant (BLES™, BLES Biochemicals, Inc. London, Ont), calfactant (Infasurf™, Forest Pharmaceuticals, St. Louis, Mo.), bovactant (Alveofact™, Thomae, Germany), bovine pulmonary surfactant (Pulmonary surfactant TA™, Tokyo Tanabe, Japan), poractant alfa (Curosurf®, Chiesi Farmaceutici SpA, Parma, Italy), and beractant (Survanta™, Abbott Laboratories, Inc., Abbott Park, Ill.)

Examples of artificial surfactants include, but are not limited to, pumactant (Alec™, Britannia Pharmaceuticals, UK), and colfosceril palmitate (Exosurf™, GlaxoSmithKline, plc, Middlesex).

Examples of reconstituted surfactants include, but are not limited to, lucinactant (Surfaxin™, Discovery Laboratories, Inc., Warrington, Pa.) and the product having the composition disclosed in Table 2 of Example 2 of WO2010/139442.

Preferably, the pulmonary surfactant is a modified natural surfactant or a reconstituted surfactant. More preferably the pulmonary surfactant is poractant alfa (Curosurf®). In another preferred embodiment, the reconstituted surfactant has composition disclosed in WO2010/139442 (see Table 2 of Example 2 of WO2010/139442).

The dose of the pulmonary surfactant to be administered varies with the size and age of the patient, as well as with the severity of the patient's condition. Those of skill in the relevant art will be readily able to determine these factors and to adjust the dosage accordingly.

Other active ingredients could advantageously be comprised in the medicament according to the invention including small chemical entities, macromolecules such as proteins, peptides, oligopeptides, polypeptides, polyamino acids nucleic acid, polynucleotides, oligo-nucleotides and high molecular weight polysaccharides, and mesenchimal stem cells derived from any tissue, in particular from a neonate tissue. In a particular embodiment, small chemical entities include those currently used for the prevention and/or treatment of neonatal respiratory diseases, for example inhaled corticosteroids such as beclometasone dipropionate and budesonide.

The method according to a preferred embodiment of the present invention exploit mathematical concepts which are common to various field of technology (e.g. hydraulic, electronic, automation and controlling theory); in the following paragraph we are providing a brief description of the basic concepts.

MATHEMATICAL DESCRIPTION OF THE SYSTEM

The rising and falling time issues are related to the intrinsic low pass filtering behavior of the system made of the atomized catheter and the compliances of the bubbles and the mechanical frame of the device.

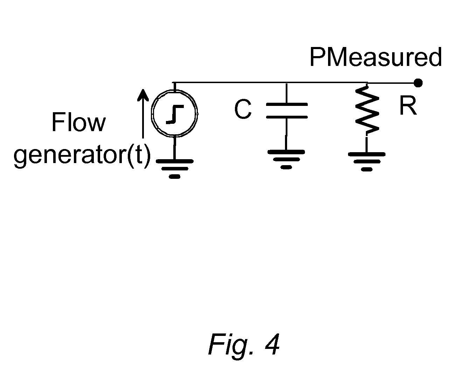

This system can be modeled as a first order system shown in Figure 4.

Where:

C = compliances of the system, mainly due to bubbles. It may be time dependent because it could change according to the amount of foam and bubbles.
R= resistance of the system, mainly due to the catheter.
Flow generator(t) = this is the action of the volumetric pump.
Pmeasured(t) = pressure measured along the surfactant line, in this case, since the only generator in the circuit is the flow generator, it is equal to the pressure generated by the pump. PinfusionPump.

This hypothesis is accurate because the system is made of: 1) the atomizing catheter, which is a long thin line filled with viscous liquid, therefore it is well approximated by a hydraulic resistance, and 2) compliances that are mainly due to bubbles and the mechanical frame. This model, according to previous experiences and testing activity is quite simple but able to describe the system, although it doesn't take into account non-linarites, that can be introduce by the infusion pump or inertial contributions, which are considered as negligible since the amount of mass of pulmonary surfactant line is minimal.

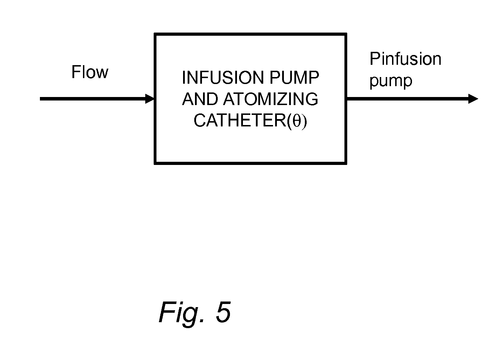

Moving from the electrical representation to a block scheme, we obtain the representation of Figure 5, where θ vector encompasses all the characteristics of the infusion pump and atomizing catheter system.

According to this implementation, the system is defined as in Equation 1,

$$\text{Equation 1} \qquad \theta=[R\ C]'$$

Getting into the Laplace domain, the system can be defined by its Laplace transformed as in Equation 2:

Equation 2 $\qquad$ *Infusion Pump and atomizing catheter*

$$= Sys1(s) = \frac{PinfusionPump}{Flow}$$

$$= \frac{R}{1 + sRC}$$

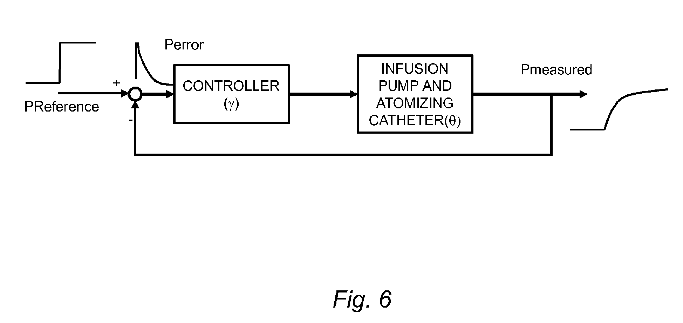

In a first aspect of the present invention, a close loop control system (Figure 6) could be applied to our device in order to minimize the rising and falling time of the pressure. The infusion pump and the atomizing catheter are fully described by a set of parameters, resumed by the $\theta$ vector; the controller is fully characterized by another set of parameters identified by the $\gamma$ vector.

Briefly, the goal is to control the motor of the infusion pump to make the pressure measured along the surfactant line more similar as possible to an ideal pressure target point selected previously and corresponding to the desired surfactant flow rate during the delivery time and zero during the hold time (i.e. during the expiratory phase).

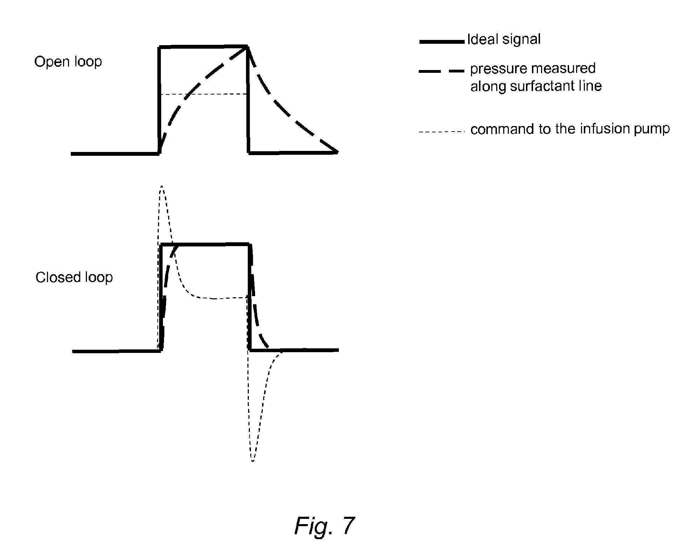

In more details, surfactant flows at a given flow rate (for instance 1.2mL/h) and it produces a certain pressure drop at the inlet of the atomizing catheter which can be measured and which is determined by Poiseville Law. The pressure drop is linearly related to the flow rate by a coefficient that is the hydraulic impedance of the system. The amount of pressure drop needed to produce the desired flow is constant unless there are big changes in the physical characteristics of the system (for example, catheter occlusion). The pressure in the catheter does not reach instantly the desired level of pressure when the motor of the infusion pump is turned on because of the compliance of the system (with "compliance of the system", we mean that behaviour that, in combination with the high resistance of the atomising catheter, introduces a loss in the performances, e.g. elastic behaviour of the mechanical frame of the infusion pump and, most importantly, gas bubbles in the surfactant circuit). If the motor is controlled by an appropriate close loop control low, in case of low time constant the controller will drive the motor to rotate faster, allowing the desired pressure in the surfactant line to be reached faster. Once the target pressure has been reached, the controller will slow down the rotation of the motor as reported in Figure 7, avoiding overshooting. The pressure sensor along the surfactant line can be used to assess the actual pressure and to regulate the velocity of the motor, accordingly to a previously defined control law with the final aim to keep the flow constant and to reduce the rising and falling times.

SIMULATION AND TEST

In order to validate the approach, simulations were performed.

**Simulation assumptions**

In a possible embodiment, the controller could be implemented by using a PIDf controller, that involves three separate constant parameters which multiplies respectively three terms which are proportional to the error (Kp), to the integral of the error (Ki) and to the derivative of the error (kd). Since the derivative is prone to enhanced noise, in this embodiment a filter was introduced on that term (Tf). Therefore the controllers are fully defined as stated in Equation 3.

Equation 3 $\qquad\qquad \gamma = [Kp \; Ki \; Kd \; Tf]'$

In the simulation, the infusion pump-atomizing catheter block is modelled as a first order system, as shown in Figure 4.

**Parameters settings**

In order to verify the test, we decided to run the test three times using the same value for resistance and flow, but changing the values for the compliances, that is representative for a different value of bubbles.

R=218*$10^9$ Pa s/m$^3$ = resistance of a catheter 18cm long and with an inner diameter of 0.8 mm filled of surfactant.

F=20*10^9 (m$^3$/s) = flow equals to 1.2 ml/min, that is the flow needed to complete the delivery in less than 15 minutes.

C = [0.5*0.9869*$10^{-12}$ 0.9869*$10^{-12}$ 2*0.9869*$10^{-12}$] m$^3$/ Pa =compliance of bubbles of 0.05, 0.1 and 0.2 mL, that is a possible amount of bubble that can be inserted unwillingly.

Therefore Preference is 4036 Pa, it represent the pressure measured in open loop at the end of transitory time once the time constant effects are done imposing the above mentioned flow in a catheter with the above mentioned resistance.

**Results**

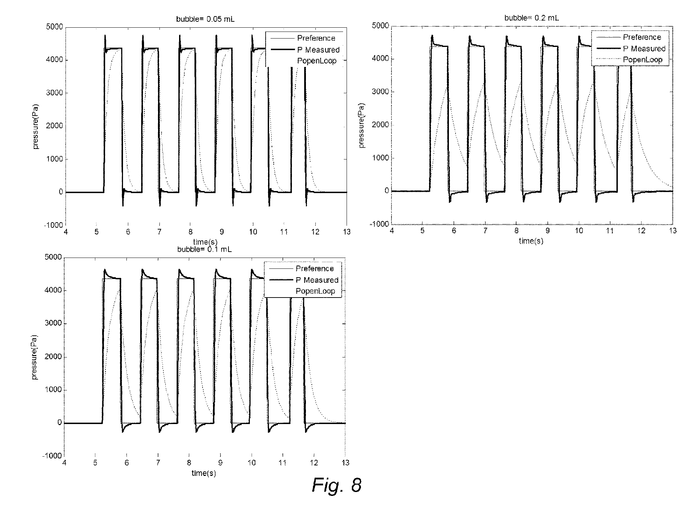

The results of the simulations, after the tuning of the PID are reported in Figure 8, where it possible to appreciate the behaviour of the system in open loop (dashed line) and closed loop (solid line) when the system is turned on/off at breathing frequency.

Some remarks about the results:

1) By an appropriate tuning of the PID it has been possible to reduce the rising time to 0.05 s despite the amount of the bubble added to the circuit has been increased.

2) The rising time without the regulator is increasing because of the addition of bubbles ranging from 0.43 s to1.72 s as the bubble increases.

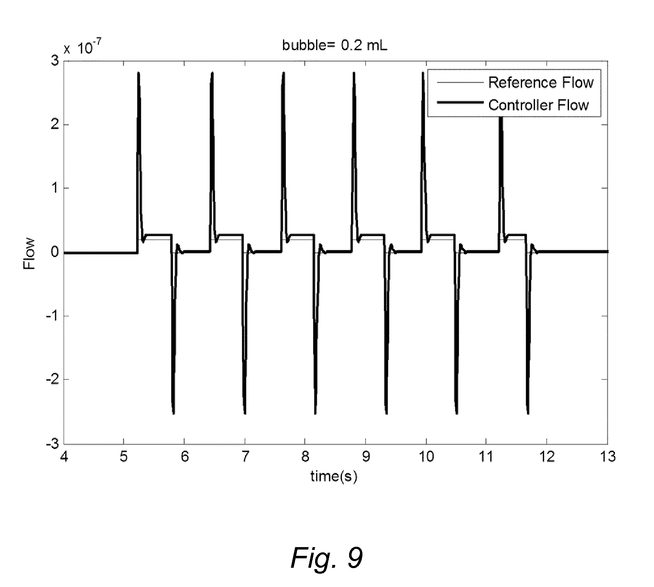

Figure 9 shows the reference flow and the flow needed to produce the pressure measured in the worst case, that is, a bubble of 0.2 mL. The maximum flow needed is just ten times the steady state flow, that means the range of velocity the infusion pump should be able to produce is quite limited and therefore not an issue from a technology perspective.

Parameters identifying the PIDf controller are reported in Table 1. Obviously they are related both to the specific implementation and to the mechanical system.

**Table 1: parameters of PIDf used as bubbles are varying.**

| Bubbles [mL] | Kp | Ki | Kd | Tf |
|---|---|---|---|---|
| 0.05 | 1,88e-11 | 2,37e-10 | -2,22e-13 | 1,17e-2 |
| 0.10 | 3,88e-11 | 3,06e-10 | -3,59e-13 | 9,24e-3 |
| 0.2 | 8,32e-11 | 3,32e-10 | -9,79e-13 | 1,17e-2 |

ALTERNATIVE EMBODIMENTS

**Embodiment 2**

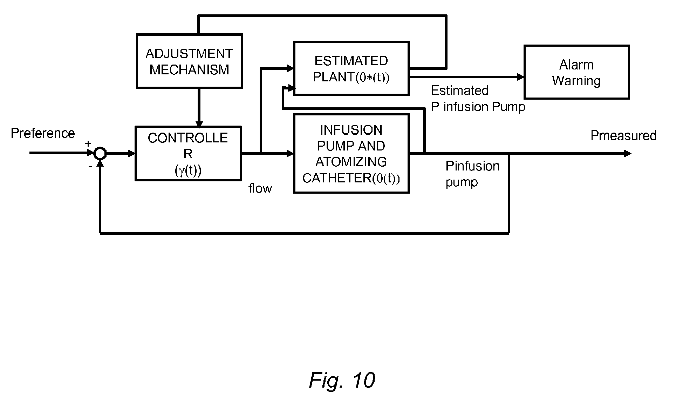

The scheme in Figure 6 reports a very general closed loop control approach in case the characteristic of the system are constant. In that condition, once the controller has been implemented, no further changes are needed. In a real case, the mechanical characteristics of the device are changing time to time because, even if the resistance of the surfactant lumen of the catheters is very reproducible, the amount of bubbles dissolved into the surfactant may change significantly and unpredictably. For this reason, the device cannot be considered as a time-independent system. Moreover, during the delivery of the surfactant, some bubbles may flow out of the catheter lowering the actual compliance of the system. As a consequence, according to a second aspect of the present invention, the scheme of Figure 6 can be updated as in Figure 10.

In this approach, the characteristic of the atomiser, which are defined by the parameters vector $\theta$, are time dependent, as enhanced by explicating the time dependence $\theta(t)$, therefore also the controller, whose characteristics are represented by the vector $\gamma(t)$, should be adapted on the base of the changes of the device characteristics. Since $\theta t)$ is unknown, it could be estimated by the response of the device, allowing to use the parameters of the estimated model $\theta^*(t)$ to tune the controller law.

The identification block allows pursuing two more functionalities:

1) Detection of the resistance in real time. Since the resistance is supposed to be constant because it is due to the dimension of the atomizing catheter, an increase may be due to an occlusion or to kinking phenomenon.

2) Estimation of the amount of bubble inserted. Immediately after the circuit has been primed the identification algorithm estimates the compliance and if it is too high, the device can warn the operator.

IN VITRO TESTING ACTIVITY

A possible embodiment of the system that allows reaching the aim of controlling the delivery of the atomization on the basis of the identified model is reported in the following paragraphs.

ATOMIZING DEVICE

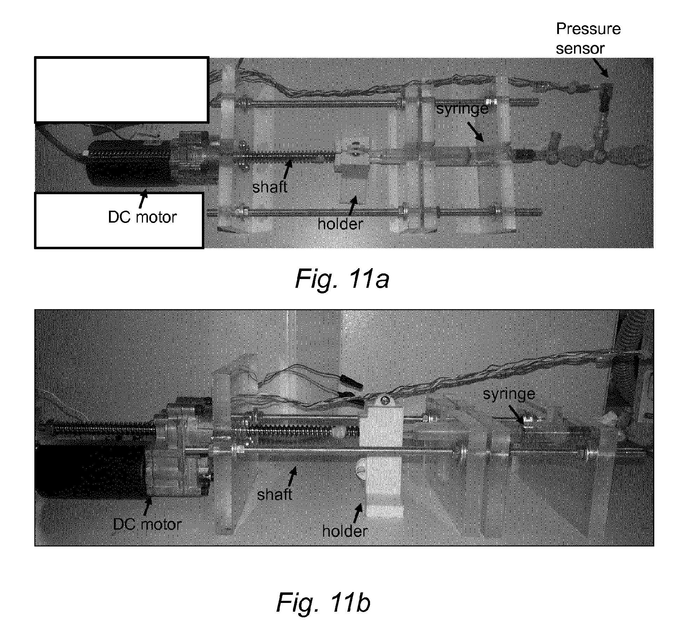

The device consists of a modified atomization device, as represented in Figure 11, made of an ad-hoc volumetric pump system (infusion pump). The system, in the present example, is made of:

1) A low cost DC motor with an encoder mounted on the rotor. The shaft of the motor is coupled with a gear to produce linear motion from the motor rotation.
2) A pressure sensor connected to the surfactant line.
3) An electronic unit comprising a) a control unit; b) a signal conditioning board for the pressure sensor and c) the driver circuits for controlling the motor.
4) A possible embodiment of the atomizing catheter as reported in PCT/EP2014/072278.
5) A gas compressor and a pressure regulator to generate and control the atomizing airflow.
6) An *ad hoc* plastic holder, developed to rigidly anchor the piston of the syringe to the shaft of the motor. Therefore, the shaft can pull and push the syringe piston.
7) Plexiglas frame developed to align the shaft to the syringe and to make the structure robust.

In this example embodiment, the syringe is filled with Curosurf® and the output of the syringe is connected to an atomizing catheter via a low-resistance/low-compliance tube. The tip of the atomizing catheter is inserted into a test lung where it senses a pressure swing similar to the one due to breathing activity. The pressure of the surfactant line is sensed at the output of the syringe by a pressure sensor.

SYSTEM IDENTIFICATION

Infusion Pump and atomizing catheter block has been described in Equation 2. Given the equation, there are several approaches for identifying the values of the parameters which may be selected on the base of the computational resources that are available and on the electronic controller unit.

A very well-known general approach is based on the Nelder-Mead Simplex Method which is able to solve any kind of minimization problem, but since our problem is linear in the parameters space, we decided to use a recursive least square algorithm (RLS).

RLS has two advantages: 1) it doesn't require high computation resources; 2) its objective function presents only a global minimum.

Recursive algorithm updated their parameter at any new given sample; therefore they are able to describe time varying models. In order to make the algorithm faster in following the variation of the parameters, we introduced also a forgetting factor.

The RLS algorithm should be able to properly identify the system even during the action of the controller, because the parameters of the controller itself rely on the state of the system and therefore they should be changes as the system changes.

PROTOCOL

The system is primed with Curosurf® trying to avoid bubbles or foam. After this, a known volume of air was inserted into the circuit to change the compliance. For each condition we:

1. Started the motor in open loop (that is without being connected to the controller), and allowed it to reach the desired target flow, 1.2mL/min;
2. Stopped the motor and waited for recovery;
3. Started the motor, with the same amount of bubbles but activating the close loop controller.

The open loop measurement is equivalent to the step response of the system. It is used to estimate the parameters by using the the Nelder-Mead Simplex Method, that we considered as the gold standard.

RLS with forgetting factor was used to estimate the same parameters during the activation of the pump in closed loop.

RESULTS

The parameters estimated by the RLS method and by the Nelder-Mead Simplex Method were compared in Table 2. They are express in arbitrary unit.

**Table 2: Comparative table**

| Bubble(ml) | Nelder-Mead Simplex Method | | RLS forgetting factor | | %error R | %error Tau | Number of repetition to converge |
|---|---|---|---|---|---|---|---|
| | R (AU) | Tau (AU) | R (AU) | Tau (AU) | | | |
| 0.1 | 0.05759 | 0.368 | 0.05708 | 0.3653 | -0,89 | -0,73 | 3 |
| 0.2 | 0.06700 | 0.6173 | 0.06721 | 0.6287 | 0,31 | 1,85 | 4 |
| 0.5 | 0.06132 | 1.432 | 0.06236 | 1.437 | 1,70 | 0,35 | 3 |
| 0.6 | 0.06570 | 1.759 | 0.06357 | 1.794 | -3,24 | 1,95 | 4 |

Remarks:

- Since the atomizing catheter is always the same, we should expect that the resistance being constant and independent from the presence and amount of bubbles. We observed a slight increase of the resistance (with both methods), likely due to some non-linearity affecting the model when high compliances values were reached.
- The resistance is estimated with a very small error, less than 4 %. The same is for the compliance that showed an error smaller than 4%.

These results suggest that the RLS method is reliable in the estimation of the model.

CONTROLLER

The control science theory deals with several strategy to optimize the control low for a dynamic system. We decided to implement the controller by means of a PID controller, whose parameters are optimized in order to make the response of the system as fast as possible and they are selected according to the amount of bubbles as reported by a look up table found empirically.

PROTOCOL

The atomizing system was the one described above.
The system is primed with Curosurf® trying to avoid bubbles or foam, although we know it is really difficult to avoid bubbles at all. Then a known volume of air is inserted into the circuit to change the compliance and a sweep is performed. A sweep consists in:

4. Starting the motor in open loop, which is without controller, and to make it reaches the desired flow, 1.2mL/min;
5. Stopping the motor and waiting for recovery;
6. Starting the motor, with the same amount of bubbles, and to control it in order to achieve the pressure target as fast as possible and to keep the pressure steady.

The amount of bubbles inserted on purpose are the one listed below, they obviously represents the amount of gas that the user might unwillingly insert.

1) 0.3 ml
2) 0.5 ml
3) 0.7 ml
4) 0.9 ml

RESULTS AND DISCUSSION

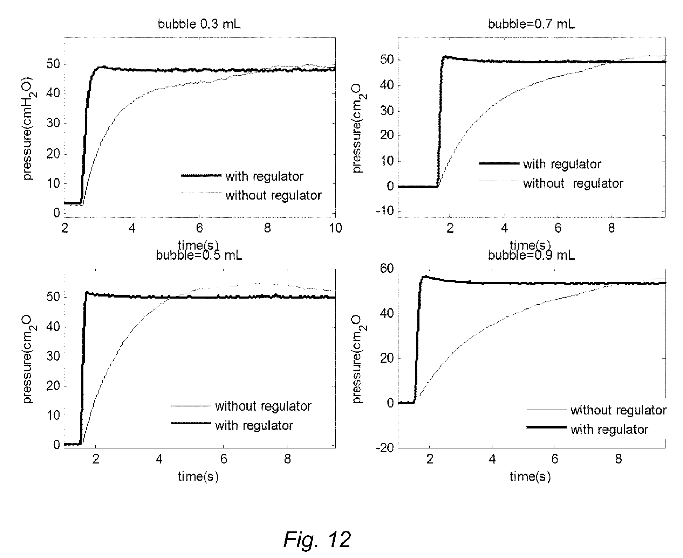

In the table below, the rising time is reported for any amount of bubbles. Rising time is defined as the time needed by the pressure to rise from 10 to 90% of the final level. Results are also represented in Figure 12.

**Table 3: bubbles inserted into the syringe and a comparison between rise time with and without the regulator.**

| Bubble (mL) | Rising time (without regulator) (s) | Rising time (with regulator) (s) |
|---|---|---|
| 0.3 | 1.57 | 0.08 |
| 0.5 | 2.35 | 0.09 |
| 0.7 | 4.90 | 0.12 |
| 0.9 | 5.38 | 0.13 |

It is clear that, by using the controller, the time needed to establish a proper atomization is shortened up to 50 times and, moreover, the region where the surfactant flow is expected to be constant is flatter as the controller acts in order to keep the system on the target values.

ADJUSTMENT MECHANISM

In this embodiment the adjustment mechanism is performed by creating a look up table that associates the parameters of the controller at the system identified. In this embodiment, the use of a table with a pre-defined number of states instead of a free self-adaptive system allows to pre-define all the possible values of the parameters and, therefore, determine all possible behaviour of the controller action. Limiting the controller action in a pre-determined range allows to warrant that the controller will always work in a safe way avoiding unpredictable behaviour of the controller that could arise when unexpected events of malfunctions affects the measured variables.
Table 4 reports the PID parameters as optimizes at any given amount of bubbles and the rising time in open and closed loop.

**Table 4: PID parameters**

| Bubble volume [ml] | Kp | Ki | Kd | Open loop [s] | Rise time-mean$\pm$std [ms] |
|---|---|---|---|---|---|
| 0.1 | 0.065 | 1.00E-04 | 0.4 | 0.957 | 44$\pm$6 |
| 0.2 | 0.095 | 1.00E-04 | 0.4 | 1.593 | 46$\pm$8 |
| 0.3 | 0.145 | 1.00E-04 | 0.6 | 2.216 | 49$\pm$7 |
| 0.4 | 0.135 | 2.00E-04 | 0.4 | 3.135 | 47$\pm$3 |
| 0.5 | 0.145 | 1.00E-04 | 0.44 | 4.026 | 81$\pm$2 |
| 0.6 | 0.155 | 2.00E-04 | 0.4 | 4.635 | 88$\pm$4 |

Obviously these parameters are tuned according to the specific mechanical frame, therefore they should be re-defined in case the structure of the infusion pump is changed.
In conclusion, thanks to this approach three goals were achieved:

1. Reduction of the rising time of the pressure signal independently of the dimension of the surfactant lumen. This allows making the design of the catheter more independent from the time constant and therefore to make the inner lumen smaller, which, in principle, may reduce the flow of the atomizing gas.
2. Reduction of the sensitivity of the system from bubbles. This will reduce the priming time since the rise time was compatible with the application even in case of large amount of bubbles.
3. Identification of the model. This will provide useful information on the system state, for instance it provides feedback about the kinking of catheter or about the degree of obstruction of the catheter.

Moreover, this approach is very effective from a cost point of view since the regulator does not require new mechanical component nor new sensors, but it needs just a firmware (or software) adjustment.

HOW TO REDUCE RISING AND FALLING TIME AND TO SYNCHRONIZE THE DELIVERY MATHEMATICAL DESCRIPTION OF THE SYSTEM

The second issues related to the delivery of atomized drug into the pharyngeal region, regards the capability of the system to identify the inspiratory phase of the breathing. This invention wants to provide new concepts that allow the delivery of surfactant and the detection of the breathing phase by using only the pressure sensor present in the surfactant line and, therefore, without needing the presence of the sensing line, with advantages in terms of costs and easiness of use.

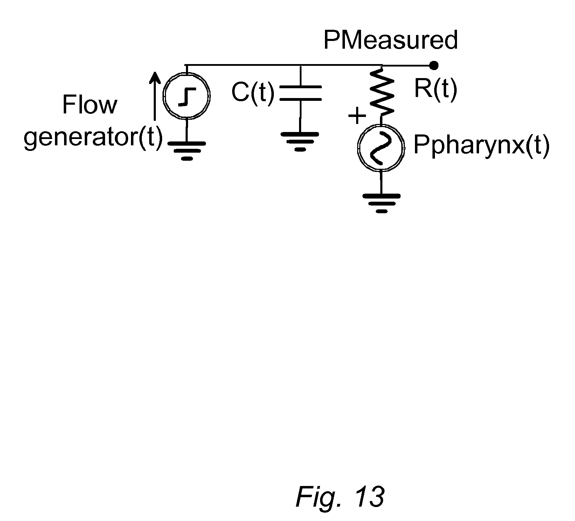

In this condition, the electrical model that describes the system is reported in Figure 13.

Please note that, in this model, a pressure generator, Ppharyngx(t), it has been added to represent the pressure that it is generated by the breathing activity in the pharyngeal region.

In Figure 13:

R and C are the same reported in Figure 4

Ppharynx(t) = pressure at the pharynx. That is the actual pressure at the pharynx.

Pmeasured(t) = pressure measured in the surfactant line: it is the combined result of the pressure developed by the breathing activity and the one resulting by the activation of the infusion pump.

In this linear model it is easy to estimate transfer functions by using the theorem of the over-imposition of the effects. By translating the problem into Laplace domain, it is possible to define the following transfer functions, as represented in Equations 5 and 6:

$$\text{Equation 5} \quad Infusion\ Pump\ and\ atomization\ catheter$$

$$= Sys1(s) = \frac{PinfusionPump}{Flow\ of\ the\ pump}$$

$$= \frac{R}{1 + sRC}$$

$$\text{Equation 6} \quad Atomizing\ catheter = Sys2(s) = \frac{Ppharynx'}{Ppharynx}$$

$$= \frac{1}{1 + sRC}$$

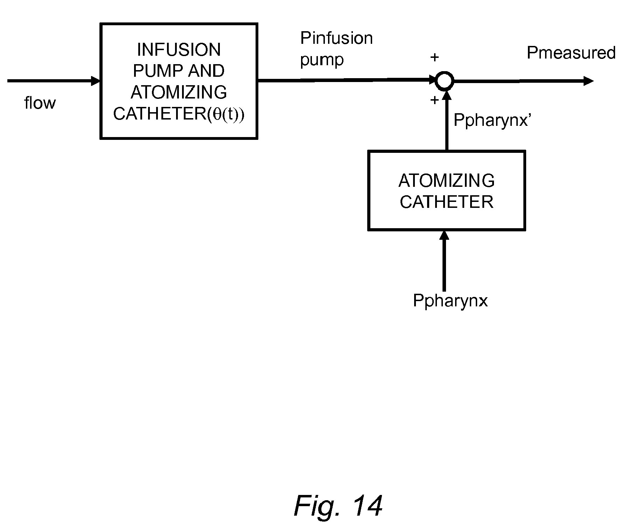

A block scheme diagram representation of this system is reported in Figure 14. Please note that, because of the low pass filtering effect of the atomizing catheter, the actual pharyngeal pressure, Ppharynx, is not directly measurable

**Embodiment 3**

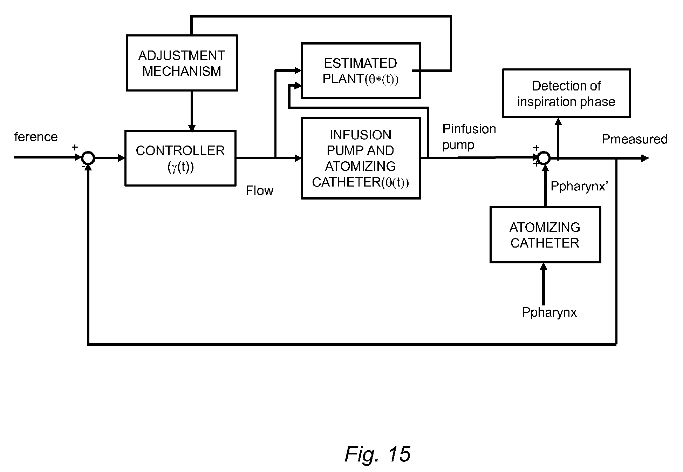

A possible approach to detect the breathing phase is the one illustrated in Figure 15: scheme of Embodiment 3.

Pmeasured is given by the sum of the contributions of the surfactant flow resulting from the intermittent activation of the infusion pump added to the pharyngeal pressure developed by the spontaneous breathing activity. As the atomizing catheter presents a high fluidodynamic resistance to the surfactant flow, the pressure generated by the infusion pump activation is usually quite bigger than the one developed by the breathing activity (in some embodiments it could be up to 10 times higher).

Embodiment 3 is based on the following consideration: during the activation of the motor, the breathing activity on the pressure signal recorded on the surfactant line can be masked by pressure drop due to the surfactant flow, conversely, when the infusion pump is not active, the breathing signal is easily detectable. Therefore, a possible solution is obtainable by detecting the beginning of the inspiration on the surfactant line (as during expiration the surfactant delivery is stopped) to start the pump activation and, as the end of expiration will be masked by the activity of the infusion pump, to stop the motor on the base of a previously estimated average inspiration time.

Expiration time can be estimated by measuring the inspiration length (Ti) and the total breathing duration (Ttot) to calculate Ti/Ttot on few spontaneous breaths of the patient done in a period in which the delivery is suspended , then it is possible to use the estimated Ti duration during the delivery phase in the following breaths in order to stop the motor at end expiration.

SIMULATION AND TEST OF EMBODIMENT 3

PROTOCOL OF EMBODIMENT 3

Data from 5 preterm infants receiving three different ventilatory supports were considered. Table 5 reports the characteristics of the patient population considered.

**Table 5: Characteristics of the patients included in the study.**

| Patient | Weight at birth [g] | Gestational age at birth [weeks+days] |
|---|---|---|
| #1 | 1415 | 32 |
| #2 | 1420 | 32 |
| #3 | 2340 | 32+4 |
| #4 | 1680 | 32+4 |
| #5 | 1690 | 31 |

On these patients, the following recordings were available:

1) Respiratory chest wall volume measured by Respiratory Inductance Plethysmography( RIP);
2) Pharyngeal pressure measured by a catheter-transducer system inserted into the pharynx of the patient.

The beginning and end of each inspiration were detected on the RIP signal in a semiautomatic way and these values were considered as reference for the comparisons.
The algorithm was run on the pharyngeal pressure signal. The assumption is that, during the activation of the motor the end of inspiration cannot be detected from the pharyngeal pressure signal. The algorithm works as a two finite states machine. As soon as one state terminates, the algorithm switches to the other state:

1) State one: breathing pattern parameters initialization state. The delivery of the treatment is suspended for few breaths (for example ten) and during this period end-expiration and end-inspiration data point are identified on the pharyngeal pressure and these data are used to estimate the average Ti/Ttot. Once the estimation of the breathing pattern parameters is concluded, the algorithm switches into the delivery state.
2) State two: delivery state. In this state, the algorithm detect the beginning of inspiration on the pharyngeal pressure signal and uses the estimated value of Ti obtained in state one to stop the infusion pump at end-expiration. As the values of Ti might change during the delivery, the delivery state runs for a pre-determined period (for example 50,100,150,200 or 250 seconds) after which the delivery is suspended and the system is switched back in state one to produce a new updated estimate of Ti.

RESULTS AND DISCUSSIONS FOR EMBODIMENT 3

Two parameters were considered to evaluate the performances of the algorithm:
• The ratio between the time of activation of the infusion pump determined by the algorithm during inspiration versus the time of the actual duration of the inspiration of the patient;
• The ratio between the time of activation of the infusion time during the expiration of the patient versus the total duration of the breath The results are reported in Table 6 (performances of the algorithm).

**Table 6**

| Initialization of breathing pattern parameters every X seconds | Percentage of surfactant delivered during an actual inspiration | Percentage of surfactant delivered during expiration (wasted) |
|---|---|---|
| X=50 | 75.0±6.2 | 5.8±2.5 |
| X=100 | 74.7±6.6 | 5.9±2.6 |
| X=150 | 73.8±8.2 | 5.9±2.7 |
| X=200 | 73.4±9.4 | 6.0±2.7 |

(continued)

| Initialization of breathing pattern parameters every X seconds | Percentage of surfactant delivered during an actual inspiration | Percentage of surfactant delivered during expiration (wasted) |
|---|---|---|
| X=250 | 72.0±9.5 | 6.0±2.6 |

Table 6 shows that the treatment was delivered incorrectly only for the 6% of the total duration of the breaths and that it was properly delivered for more than 70% (in average) of the duration of the inspirations even in the worst case of X equals to 250 s.

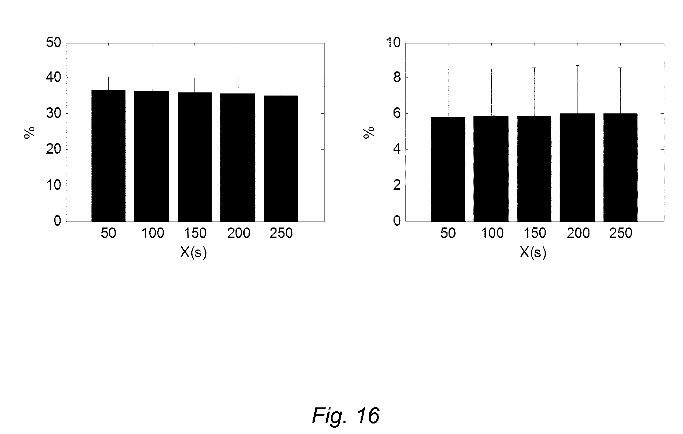

The data reported seems to state that the values of Ti estimated in the state one is quite stable with time (Figure 16). Statistical analysis was performed to clarify this aspect by one-way ANOVA test. Between the population no statistical difference was found (p<0.0001) as expected. Also surfactant delivery during expiration didn't show a dependence on the interval between initialization phase (p<0.0001), at least if it ranges from 50 to 250 seconds.

IN VITRO TESTING ACTIVITY FOR EMBODIMENT 3

TESTING ACTIVITY

SET UP AND PROTOCOL

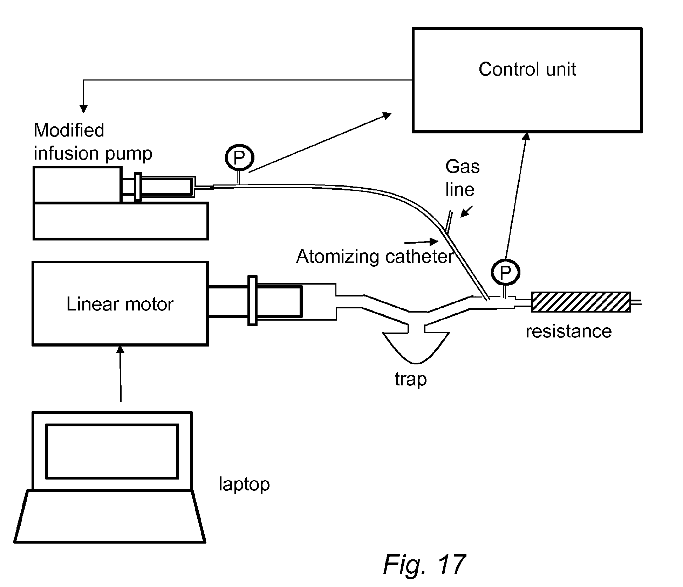

The bench test system (Figure 17) consisted of a modified atomizing device and a pressure generator able to reproduce the actual pressure signal at the pharynx. The pressure generator produced a pressure signal equals to the actual pharyngeal pressure measured on a preterm infant in a chamber. The pressure in that chamber is sensed at the same time by the atomizing catheter and by a second pressure sensor used as gold standard for further analysis.

In order to test the capabilities of the breathing detection algorithm, the possible embodiment of the atomized device described above was used. The pressure generator consisted of a servo controlled linear motor whose shaft is connected to the piston of a syringe; the motion of the motor produces a flow that is linearly related to its velocity, the flow is then producing a pressure, which is our aim, by adding an hydraulic resistance in series to the line. The pressure generated is equal to the flow times the resistance. The trap represented in Figure 17 collects the atomized liquid preventing the medicament from entering in contact with the motor/syringe embodiment.

Once the infusion pump is loaded with surfactant, the synchronisation algorithm is started. The beginning of the inspiration is detected on the pressure signal, while the beginning of expiration is based on the Ti/tot ratio estimated during the initialization state, as described above. Since simulation tests assessed that the interval between one initialization state and the following one is not a very sensitive parameter, in this experiment it has been set to 250 seconds.

RESULTS FOR EMBODIMENT 3

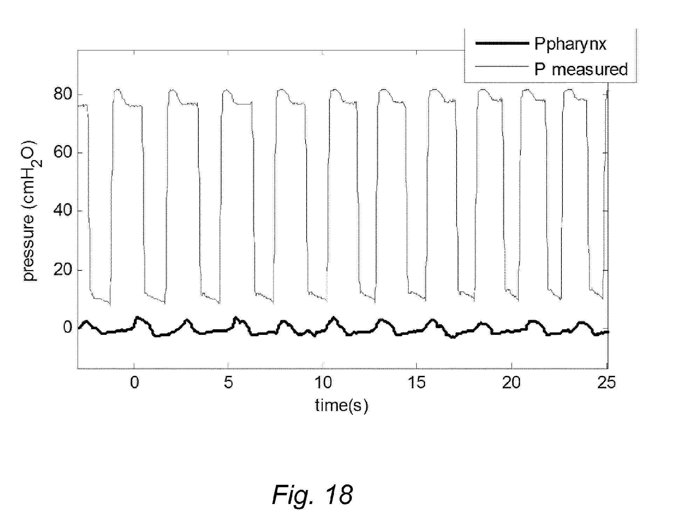

Figure 18 shows qualitative results. It can be noted that:

1) The synchronization between the activation of the motor and the beginning of the inspiration is quite responsive;
2) The system shows a very fast rising and falling time (approx.. 0.05 s);
3) The system shows very reproducible pressure swings into the surfactant line, suggesting a quite fine control on the surfactant delivered.

Even if this approach is able to provide an effective solution for many applications, there are two possible limitations that may occur: 1) If the internal lumen of the surfactant channel in the atomising catheter is very small, there is a possible phase shift introduced to the transmission of the pharyngeal pressure signal through the atomizing catheter and 2) if the newborn shows a highly variable breathing pattern or if he/she changes the breathing pattern as a consequence of the treatment, the possible errors in start and stop of the delivery might become more relevant.

Concerning the first issue, by measuring the pressure into the medicament channel of the system, the device measures Ppharynx' which is the filtered version of the actual Pharynx according to Equation 7. The filter modifies the original signal by reducing the amplitude of high frequency components but also introducing a delay that depends on the frequency.

For instance, in case the time constant is 0.43s, the pole of the system is at 0.37 Hz, if the breathing frequency is 1 Hz, the Ppharynx' signal is delayed of 0.25 s compared to Ppharynx. This delay introduces a big synchronization error. In our invention it is possible to prevent the error thanks to the identification of the parameters that characterises the mechanical behaviour of the system.

The second issue may happen in case of irregular breathing pattern. In fact, since the end of expiration of each single breath is not actually detected but it is estimated on previous breathings, there could be relevant differences between the estimated value and the actual one in case of very irregular and time varying breathing pattern, leading to surfactant waste.

Embodiment 4 discloses a solution that allows detecting both end inspiration and end expiration. Embodiment 5 describes a possible solution that allows addressing the issues associated to the delay in the transmission of the signal.

**Embodiment 4**

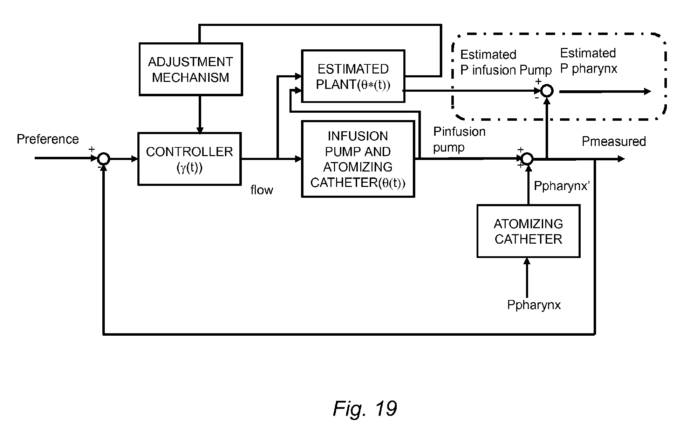

The complete working scheme of Embodiment 4 is reported in Figure 19, where the differences compared to Embodiment 2 are enclosed within a dashed line. The controller action is acting to make the response of the system as similar as possible to the reference pressure, Preference, that is a step-like signal equals to the flow times the hydraulic resistance of the catheter.

Since the measured pressure, Pmeasured, contains also the contribution of the breathing activity, the controller action tries to compensate even for that signal, thus the controller output will contain informative content about Ppharynx'. If the infusion pump and atomizing catheter block and the estimated plant block are fed with the same signal, i.e. the output of the controller, the estimated P infusion Pump and Pmeasured will be different. This is because the output of the estimated system doesn't include the breathing activity but just the response of a fist order system to the controller action. Then, by subtracting the estimated signal to the actual measured pressure, we can obtain the respiratory signal.

SIMULATION AND TEST FOR EMBODIMENT 4

Simulations were run to test this embodiment. The following assumptions were considered:

1) the simulation requires to describe both the actual delivery system made of "infusion pump and the atomizing catheter", as reported in Figure 19, and the system that is estimated from the recorded pressure and flow, "estimated plant" as reported in Figure 19, whose parameters are identified on line continuously. As found in vitro testing of embodiment 2, the identification process introduce an average error on the estimation of the parameters that is around 5%, therefore an error of 5% was added to the parameters of the "estimate plant", respect to the parameter of .the actual "infusion pump and the atomizing catheter".

2) "Controller", as reported in Figure 19, has been chosen from PID family and it has been design on order to reach a rising and falling time of 50 ms as obtained in the in vitro testing of embodiment 2.

3) Preferably, figure 19, represents the ideal pressure signal recorder on the surfactant line, in case the system had no compliance at all. It is a step signal.

SETTING OF PARAMETERS AND INPUTS FOR EMBODIMENT 4

The value used for the simulations are:

Simulation 1

R=218*109 Pa s/m3 = resistance of a catheter 18cm long and with an inner diameter of 0.8 mm filled of surfactant

C=0.9869*10-12 m3 / Pa =compliance of a bubble 0.1 mL, that is a possible amount of bubble that can be inserted unwillingly

F=20*10^-9 (m3/s) = flow equals to 1.2 ml/min, that is the flow needed to complete the delivery in less than 15 minutes.

Simulation 2

The same values were used but the compliance which was doubled: C=2*0.9869*10-12 m3 / Pa =compliance of a bubble 0.2 mL, that is a possible amount of bubble that can be inserted unwillingly

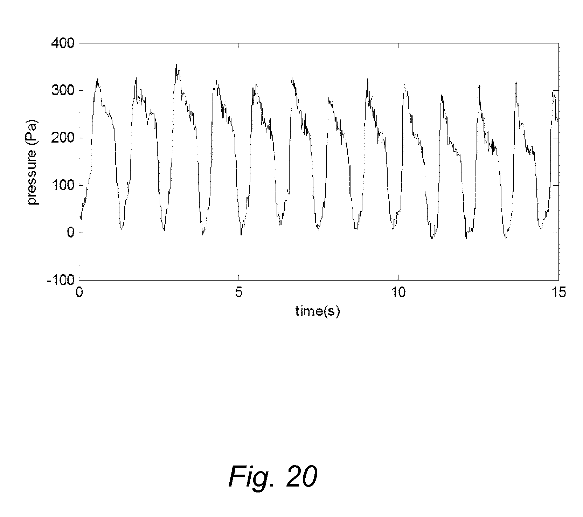

Ppharynx used in both simulations is the pharyngeal pressure measured in a term piglet breathing spontaneously under CPAP. The piglet weights 1 kg. This could be considered a good reference for the baby breathing pattern, Figure 20.

RESULTS FOR EMBODIMENT 4

Rising and falling time

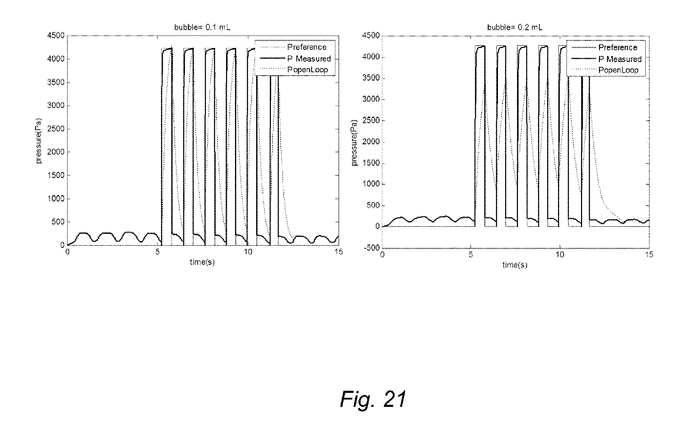

Figure 21 shows the rising and falling time obtained with two different amounts of air bubbles. In the same figure, the open loop response of the system is reported too. It is interesting to notice that with a total amount of gas bubbles equals to 0.1 mL, the amount of surfactant delivered during expiration (that is wasted) is comparable to that delivered during inspiration. In case of more bubbles, 0.2 mL, the system cannot work properly and the surfactant is delivered continuously, even during expiration.

Detection of pharyngeal pressure

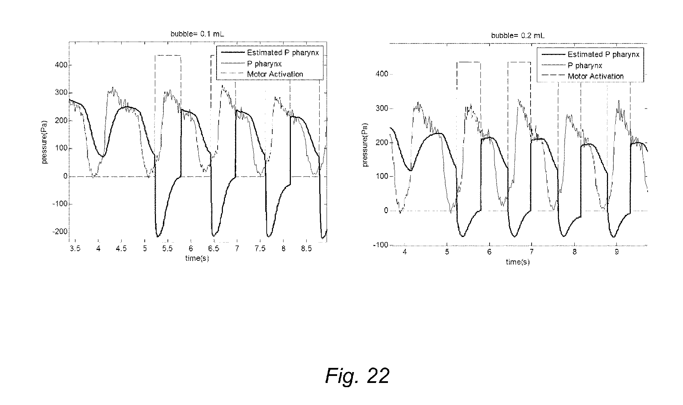

In Figure 22 the estimated pharynx pressure signal is reported and compared to the actual one. Although the strong low pass effect and the abrupt changes as the motor was turned on and off, it is still possible to identify the breathing phase. Possible limitations to this approach are related to the delay added by the surfactant line on the reconstructed pharyngeal pressure as for Embodiment 4. Embodiment 5 presents an approach that is able to compensate for the delay on the surfactant line. Part of this approach can be applied even to Embodiment 4.

**Embodiment 5**

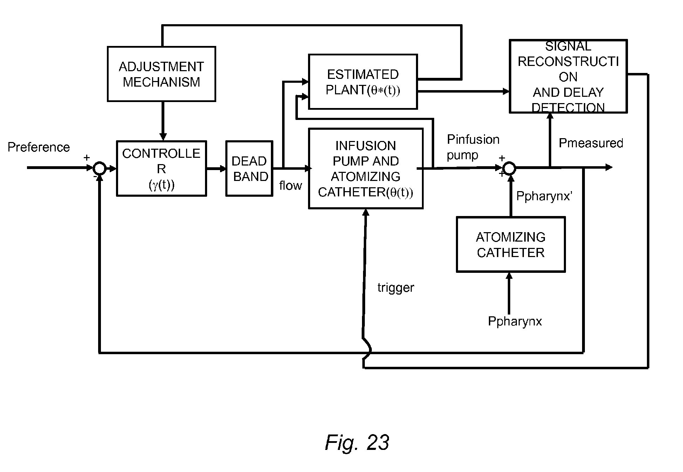

If the time constant of the system is particularly high, that means the delay between the actual pharyngeal pressure, Ppharynx, and the recorded one, Ppharynx', is too high the afore mentioned concepts could present some limitations that are overcome by Embodiment 5, Figure 23.
Scheme of Embodiment 5 differs from Embodiment 3, Figure 15, because of two blocks:

  1) Dead band
  2) Signal reconstruction and delay detection.

The purpose of these blocks are discussed in detail here below:

DEAD BAND

In the previous concepts, the controller action has two aims: to compensate for the time constant and to compensate for the effect of the breathing activity that is removed from the signal measured, Pmeasured.
The insertion of the dead band block makes the controller able to compensate only for the time constant effects and not for the breathing activity. This result is obtained by setting the resolution used by the controller to change the flow bigger than the amplitude of the breathing signal, which is several times smaller than the activation signal. In other words, the changes in the measured pressure due to the breathing activity, are under threshold to be considered by the controller and does not results in actions on the infusion pump.
As a finale result, which is the aim in inserting the dead band, Pmeasured contains the breathing activity both when the motor is activated under the controller action and both when the motor is turned off.

SIGNAL RECONSTRUCTION AND DETECTION OF THE PHASE DELAY

Signal reconstruction

Thanks to dead band block, the breathing signal is always detectable on Pmeasured except during the transition of the motor from on to off and vice versa. Since the pressure step in these phases is known, it is possible to fully reconstruct the signal by removing it from PReference. This will result in some blanking time limited to rising and falling time but, if the controller works properly, they will be limited to few milliseconds.

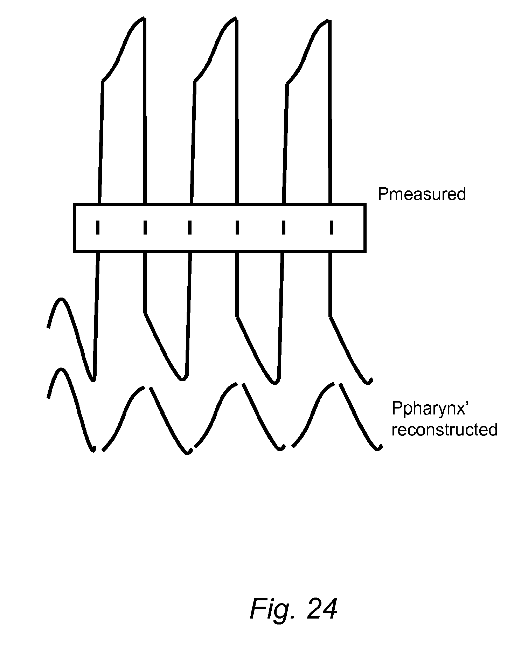

Figure 24 shows this concept: top) Pmeasured contains breathing activity, bottom) the pharyngeal pressure reconstructed by removing the activation signal due to the motor activation.

Detection of delay in the sensing signal

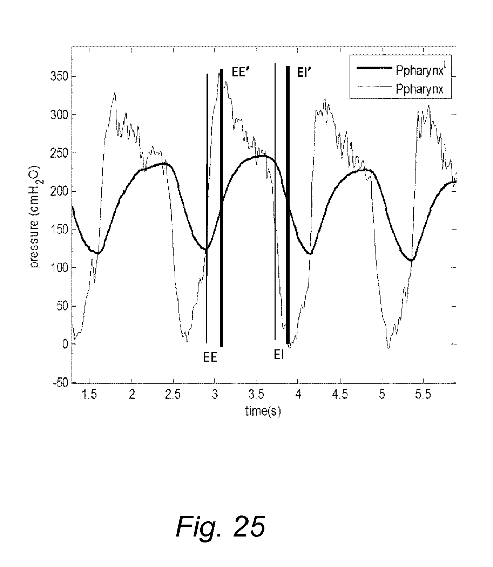

Although dead band allows reconstructing the breathing signal, there is still the issue associated to the delay added by the mechanical characteristics of the catheter (Figure 25).
The actual end of expiration, EE, is delayed compared to the measured one, EE', that means the delivery of the drug would not be in phase with the actual breathing but it would be delayed of a time that depends on the time constant of the system and the breathing frequencies and can be calculated by Equation 7, where Sys 2 is the block representing the atomizing catheter as in Equation 6 and $T_{breathing}$ is the inverse of the respiratory rate.

$$\text{Equation 7} \qquad T_{delay} = T_{breathing} * \frac{\measuredangle Sys2\left(2\pi\frac{1}{T_{breahing}}\right)}{2\pi}$$

TRIGGER SIGNAL FOR EMBODIMENT 5

In order to work properly, the atomising system should be promptly triggered at the beginning of inspiration. To reach this aim, all the elements described above have to be combined together:

1) The reconstructed signal is delayed of a time, $T_{delay}$, that could be estimated by means of the estimated parameters of the system.
2) As shown above, with reference to Embodiment 3, given the pharyngeal pressure signal it is possible to predict with a good accuracy the duration of the next inspiration and the next expiration.

Therefore, once the end inspiration on the reconstructed signal has been detected, the next activation of the motor, $T_{trigger}$, Equation 8, would happen after a time equals to the mean duration of the expiration, $T_{expiration'}$, corrected by the delay introduced by the surfactant line, $T_{delay}$ as estimated in Equation 7.

$$\text{Equation 8} \qquad T_{trigger} = T_{expiration'} - T_{delay}$$

IN VITRO TESTING ACTIVITY

The aim of the in vitro testing activity is to prove the feasibility of the approach that can be inferred by the capability of the system to reconstruct the pharyngeal pressure.

Set up

The set up was the same reported for Embodiment 3. The amount of bubble added is 0.2 mL.

SIGNAL RECONSTRUCTION

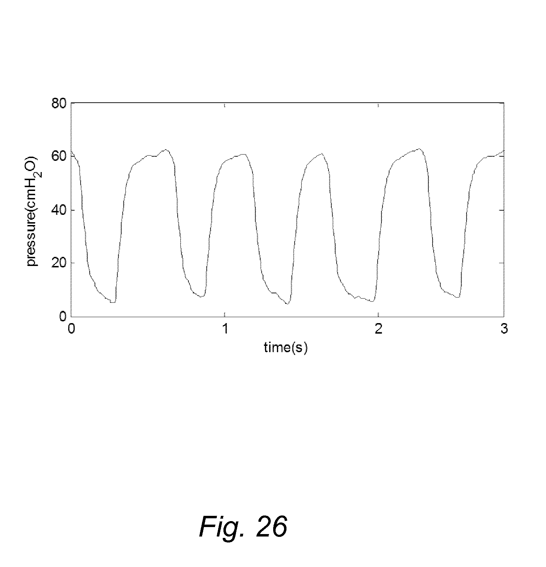

Figure 26 shows the pressure measured along the surfactant line during start and stop of the motor. In this embodiment, the controller has been tuned to minimize extra-shootings in the pressure signal even though this has been slightly increased the rising and falling times. Breathing activity is still difficult to detect because it is partially masked by the turning on and off of the motor.

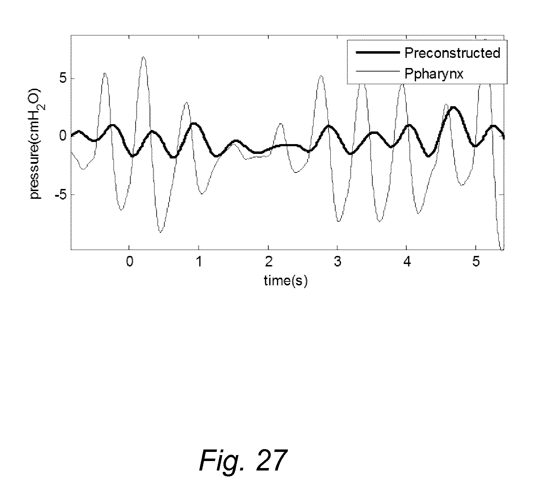

Figure 27 shows the reconstructed signal compared to the actual pressure. They present a similar trend and although the reconstructed signal has a slightly different shape compared to the actual one, end inspirations and expirations are well detectable.
Advantageously, the system of the invention is applied to pre-term neonates who are spontaneously breathing, and preferably to extremely low birth weight (ELBW), very-low-birth-weight (VLBW), and low-birth weight (LBW) neonates of 24-35 weeks gestational age, showing early signs of respiratory distress syndrome as indicated either by clinical signs and/or supplemental oxygen demand (fraction of inspired oxygen ($FiO_2$) > 30%).
As non-invasive respiratory support, in a preferred embodiment, nasal Continuous Positive Airway Pressure (nCPAP) could be applied to said neonates, according to procedures known to the person skilled in the art. Preferably a nasal mask or nasal prongs are utilised. Any nasal mask commercially available may be used, for example those provided by The CPAP Store LLC, and the CPAP Company.
Nasal CPAP is typically applied at a pressure comprised between 1 and 12 cm water, preferably 2 and 8 cm water, although the pressure can vary depending on the neonate age and the pulmonary condition.
In another preferred embodiment, nasal intermittent positive-pressure ventilation (NIPPV) could be applied.
Other non-invasive ventilation procedures such as High Heated Humidified Flow Nasal Cannula (HHHFNC) and bi-level positive airway pressure (BiPAP) could alternatively be applied to the neonates.

**Claims**

1.  A system for delivering a liquid medicament to spontaneously breathing patients, comprising:

    - i) a catheter adapted to reach the pharyngeal region of the patient, the catheter including at least a first channel being adapted to convey in the patient's pharyngeal region a flow of liquid medicament,
    - ii) first pump means connected to a first end of the at least first channel, adapted to create a pressure which pushes the column of liquid medicament towards the second end of the at least first channel;
    - iii) breathing detecting means, for measuring a value indicative of whether the patient is in an inspiration or in an expiration phase and wherein the first pump means are controlled by a microprocessor and are selectively activated only during inspiration phase, **characterized in that** the pressure applied to the first pump means is adapted by the microprocessor so that the delay induced by the hydraulic impedance of the system is substantially counterbalanced.

2.  The system of claim 1, wherein the medicament comprises a pulmonary surfactant.

3.  The system of claim 1 or 2, wherein the delay induced by the hydraulic impedance is counterbalanced by increasing the initial pressure of the first pump means so that the pressure drop caused by the hydraulic impedance is avoided.

4.  The system of any preceding claim, wherein breathing detecting means includes pressure detective means, for measuring a value indicative of the pressure in the patient pharyngeal cavity, such value being used to determine whether the patient is in an inspiration or in an expiration phase.

5.  The system of claim 4, wherein the pressure detecting means includes:

    iv) a pressure sensor connected to the first channel for measuring a value indicative of the pressure on the flow of the liquid medicament..

6.  The system of claim 4, wherein the pressure detecting means includes:

    iv) a second channel adapted to convey in the patients pharyngeal region a flow of a water solution;
    v) second pump means connected to a first end of the second channel, adapted to create the flow of water solution;
    vi) a pressure sensor connected to the second channel for measuring a value indicative of the pressure on the flow of water solution.

7.  The system of claim 4, 5 or 6, wherein the pressure detecting means is connected and provides feedback to the microprocessor and wherein the microprocessor estimates the hydraulic impedance according to measured values received from the pressure detecting means.

8.  The system of any claim 4 to 7, wherein the hydraulic impedance is calculated as a function of the difference between the measured value received from the pressure detecting means and a predetermined expected value.

9.  The system of any claim 3 to 8 wherein the increase of the initial pressure is determined according to predetermined values.

10. The system of claim 9 wherein the predetermined values are stored in a lookup table accessible by the microprocessor.

11. The system of any preceding claim, wherein the catheter includes at least a dedicated gas channel adapted to convey in the patient's pharyngeal region a pressurized flow of gas, the system further comprising:

    - gas pump means connected to a first end of the gas channel, adapted to create the flow of pressurized gas;

    so that when the column of liquid medicament and the pressurized gas meet in the pharyngeal cavity, the liquid column is broken into a plurality of particles causing the atomized medicament to be delivered into the patient's lungs.

PRESSURE
SENSOR

Modified
infusion
pump

piston

syringe
piston

*Prior Art*

*Fig. 1*

*Fig. 2a*                                                         *Fig. 2b*

Fig. 3

*Fig. 4*

Flow → **INFUSION PUMP AND ATOMIZING CATHETER($\theta$)** → Pinfusion pump

*Fig. 5*

Fig. 6

Open loop

Closed loop

Ideal signal

— — pressure measured
along surfactant line

------- command to the infusion pump

*Fig. 7*

Fig. 8

*Fig. 9*

*Fig. 10*

Fig. 11a

Fig. 11b

Fig. 12

*Fig. 13*

INFUSION
PUMP AND
ATOMIZING
CATHETER(θ(t))

flow

Pinfusion
pump

+

Pmeasured

+

Ppharynx'

ATOMIZING
CATHETER

Ppharynx

*Fig. 14*

*Fig. 15*

*Fig. 16*

Control unit

Modified
infusion pump

Ⓟ

Gas
line

Atomizing catheter

Linear motor

Ⓟ

resistance

trap

laptop

*Fig. 17*

*Fig. 18*

*Fig. 19*

*Fig. 20*

*Fig. 21*

*Fig. 22*

*Fig. 23*

Pmeasured

Ppharynx'
reconstructed

*Fig. 24*

*Fig. 25*

*Fig. 26*

*Fig. 27*

Europäisches
Patentamt

European
Patent Office

Office européen
des brevets

# EUROPEAN SEARCH REPORT

Application Number

EP 15 17 2198

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2013/160129 A1 (CHIESI FARMA SPA [IT]; MILANO POLITECNICO [IT]) 31 October 2013 (2013-10-31) * the whole document * | 1-11 | INV. A61B5/087 A61M15/00 A61M15/08 A61M16/00 A61M16/04 |
| X | US 2005/229926 A1 (FINK JAMES [US] ET AL) 20 October 2005 (2005-10-20) * figures 1-6 * * paragraph [0034] - paragraph [0068] * | 1-11 | |
| X | EP 0 692 273 A1 (TRUDELL MEDICAL LTD [CA]) 17 January 1996 (1996-01-17) * the whole document * | 1-11 | |
| A | EP 1 818 070 A2 (PARI GMBH [DE]) 15 August 2007 (2007-08-15) * the whole document * | 1-11 | |
| A | EP 0 758 257 A1 (BRAUNER MARK [US]) 19 February 1997 (1997-02-19) * the whole document * | 1-11 | |

TECHNICAL FIELDS
SEARCHED      (IPC)

A61M

The present search report has been drawn up for all claims

| Place of search | Date of completion of the search | Examiner |
|---|---|---|
| Munich | 11 November 2015 | Liess, Helmar |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
.....................................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 15 17 2198

This annex lists the patent family members relating to the patent documents cited in the above-mentioned European search report.
The members are as contained in the European Patent Office EDP file on
The European Patent Office is in no way liable for these particulars which are merely given for the purpose of information.

11-11-2015

| Patent document cited in search report | | Publication date | Patent family member(s) | | Publication date |
|---|---|---|---|---|---|
| WO 2013160129 | A1 | 31-10-2013 | AR | 090811 A1 | 10-12-2014 |
| | | | AU | 2013251865 A1 | 06-11-2014 |
| | | | CA | 2870391 A1 | 31-10-2013 |
| | | | CL | 2014002863 A1 | 10-04-2015 |
| | | | CN | 104321103 A | 28-01-2015 |
| | | | CO | 7160044 A2 | 15-01-2015 |
| | | | EA | 201491812 A1 | 27-02-2015 |
| | | | EP | 2841135 A1 | 04-03-2015 |
| | | | JP | 2015514481 A | 21-05-2015 |
| | | | KR | 20150004832 A | 13-01-2015 |
| | | | PE | 06042015 A1 | 07-06-2015 |
| | | | PH | 12014502321 A1 | 12-01-2015 |
| | | | US | 2013333695 A1 | 19-12-2013 |
| | | | WO | 2013160129 A1 | 31-10-2013 |
| US 2005229926 | A1 | 20-10-2005 | HK | 1105174 A1 | 30-11-2012 |
| | | | US | 2005229926 A1 | 20-10-2005 |
| | | | US | 2005229929 A1 | 20-10-2005 |
| EP 0692273 | A1 | 17-01-1996 | AT | 263595 T | 15-04-2004 |
| | | | AT | 527009 T | 15-10-2011 |
| | | | AU | 700225 B2 | 24-12-1998 |
| | | | AU | 2169995 A | 04-01-1996 |
| | | | BR | 9502836 A | 06-02-1996 |
| | | | CA | 2152002 A1 | 18-12-1995 |
| | | | DE | 69532843 D1 | 13-05-2004 |
| | | | DE | 69532843 T2 | 28-04-2005 |
| | | | EP | 0692273 A1 | 17-01-1996 |
| | | | EP | 1433493 A2 | 30-06-2004 |
| | | | JP | 3746090 B2 | 15-02-2006 |
| | | | JP | 3869412 B2 | 17-01-2007 |
| | | | JP | H0838607 A | 13-02-1996 |
| | | | JP | 2004081890 A | 18-03-2004 |
| | | | JP | 2006289116 A | 26-10-2006 |
| | | | NZ | 272354 A | 24-10-1997 |
| | | | RU | 2141351 C1 | 20-11-1999 |
| | | | US | 5964223 A | 12-10-1999 |
| | | | US | 6526976 B1 | 04-03-2003 |
| | | | US | 2009107503 A1 | 30-04-2009 |
| | | | ZA | 9504936 A | 27-02-1996 |
| EP 1818070 | A2 | 15-08-2007 | DE | 102006006183 A1 | 16-08-2007 |
| | | | EP | 1818070 A2 | 15-08-2007 |
| | | | EP | 2567724 A2 | 13-03-2013 |
| | | | US | 2008000470 A1 | 03-01-2008 |
| | | | US | 2015174344 A1 | 25-06-2015 |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

EP 15 17 2198

This annex lists the patent family members relating to the patent documents cited in the above-mentioned European search report.
The members are as contained in the European Patent Office EDP file on
The European Patent Office is in no way liable for these particulars which are merely given for the purpose of information.

11-11-2015

| Patent document cited in search report | Publication date | Patent family member(s) | Publication date |
|---|---|---|---|
| EP 0758257 A1 | 19-02-1997 | CA 2190196 A1<br>EP 0758257 A1<br>WO 9530448 A1 | 13-05-1998<br>19-02-1997<br>16-11-1995 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013160129 A
- EP 2014072278 W
- WO 9532992 A
- WO 8906657 A
- WO 9222315 A
- WO 0047623 A
- WO 2010139442 A

**Non-patent literature cited in the description**

- **WAGNER MH ; AMTHAUER H ; SONNTAG J ; DRENK F ; EICHSTÄDT HW ; OBLADEN M.** Endotracheal surfactant atomization: an alternative to bolus instillation?. *Crit Care Med.,* 2000, vol. 28 (7), 2540